# EUROPEAN PATENT SPECIFICATION

(11) **EP 1 140 798 B1**
(45) Date of publication and mention of the grant of the patent: **14.05.2003**
(21) Application number: 99974135.8
(22) Date of filing: 28.10.1999
(51) Int. Cl.: C07C 231/12, C07C 233/36, C07C 233/38

(54) **PROCESS FOR THE PREPARATION OF FLOWABLE CONCENTRATED BETAINE SOLUTION**
VERFAHREN ZUR HERSTELLUNG FLIESSFÄHIGER, KONZENTRIERTER BETAINLÖSUNGEN
PROCEDE DE PREPARATION DE SOLUTION DE BETAINE CONCENTREE ET FLUIDE

(43) Date of publication of application: 10.10.2001
(73) Proprietor: EOC Surfactants NV, 9940 Evergem (BE)
(72) Inventor: DE RIJCKE, Daisy, B-9690 Kluisbergen (BE); TANGHE, Stijn, B-8700 Aarsele (BE); STAELENS, Pascal, B-1790 Affligem (BE); DELAHAYE, Peter, B-9750 Ouwegem (BE)
(74) Representative: Powis de Tenbossche, Roland
(86) International application number: BE9900138
(87) International publication number: WO01030744

(56) References cited:
- EP-A- 0 560 114
- EP-A- 0 656 346
- WO-A-95/24377
- DE-C- 4 408 183

## Description

The present invention relates to a process for the preparation of a flowable aqueous solution of betaine(s) of the general formula : 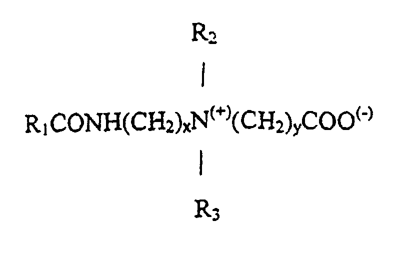 wherein
R₁ is the alkyl portion of a fatty acid with 6 to 24, preferably from 6 to 18 carbon atoms ;
R₂ and R₃ are the same or different and represent alkyl with 1 to 4 carbon atoms ;
x is an integer from 1 to 5, preferably an integer equal to 2 or 3, and
y is 1,2 or 3,
in which a fatty acid amide of formula

R₁CONH(CH₂)ₓNR₂R₃

is quaternized with at least a ω-halogenoalkylcarboxylic acid of formula X(CH₂)_{y}COOH with X a halogen atom or a salt thereof, at least partly in an aqueous medium having a pH greater than 4 and a solid content lower than 90%.

Example 1 of US 4,861,517 discloses the preparation of an aqueous solution of betaine having the above formula. Said aqueous solution has a pH of 5 and a solid content of 42% and is liquid. When the said liquid solution is concentrated upto a solid content of 43-44%, a solid gel is obtained.

Examples 2 and 3 of US 4,861,517 discloses a specific preparation process for obtaining an aqueous betaine solution with a solid content of about 47% which is liquid at room temperature. However, as soon as the temperature is lowered, a gel is formed.

In order to solve the problem, US 4,861,517 suggests to reduce the pH of the aqueous solution to less than 4.5 by adding a mineral acid such as hydrochlorhydric acid and sulfuric acid.

EP 0 560 114 discloses a process for the preparation of a liquid aqueous betaine solution. According to said document, the betaine solution having a pH between 5 and 8 and a solid content of at least 40% (especially of at least 45%) is liquid due to the presence of 1 to 3% by weight of free fatty acid.

When repeating the process of EP 0 560 114, it appears that betaine solutions with a solid content higher than 45% and with a free fatty acid content of about 1.2 % by weight are not liquid at room temperature. This fact is confirmed by example A3 of EP 560 114, as well by the teaching of WO95/24377 and PCT/JP97/04024 (the contents of which are incorporated by reference). According to PCT/JP97/04024 (WO 98/20093), the fluidizing effect of the free fatty acids is rather limited, which creates manufacturing and stability problem and makes it impossible to achieve compositions with good fluidity when a concentration of 50% dry residue or slid content is desired. In order to solve this problem, WO95/24377 proposes the use of specific additives together during the quaternization reaction, such as a mixture of non ionic surfactant and polyols. In order to obtain liquid betaine solution with a solid content of more than 50%, a large amount of non ionic surfactant is used in WO95/24377. WO 98/20093 proposes the use of specific dimers and esters of fatty alcohols or polyoxyethylated fatty alcohols.

It has now been discovered that when using during the quaternization reaction or a part thereof, a compound of formula 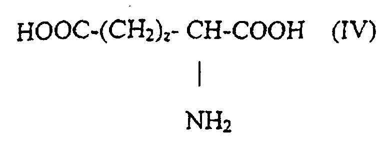 with z an integer from 1 to 10
or a salt thereof or the product of reaction thereof (i.e. product of the reaction of an acid of formula IV or product of the reaction of a salt of an acid of formula IV) with a ω-halogenoalkylcarboxylic acid of formula X(CH₂)_{y}COOH with X a halogen atom or a salt thereof, it was possible to obtain an aqueous betaine solution with a solid content higher than 45% or an aqueous betaine solution concentrated to a solid content higher than 45% which is flowable at a temperature of less than 10°C, preferably of less than 5°C. Already, excellent result can be obtained when using very small amount of said compound in the reaction mixture. For example, the weight ratio betaine/ additive of formula IV is comprised between 150/1 and 10/1, advantageously between 100/1 and 20/1, preferably between 80/1 and 40/1

Preferably, the quaternization reaction is carried out in presence of a reaction product of an acid of formula IV or a salt of said acid of formula IV, with a ω-halogenoalkylcarboxylic acid of formula X(CH₂)_{y}COOH with X a halogen atom or a salt thereof. Advantageously, the said reaction product is the product of the reaction in an aqueous medium of an acid of formula IV or a salt of said acid of formula IV, with a ω-halogenoalkylcarboxylic acid of formula X(CH₂)_{y}COOH with X a halogen atom or a salt thereof.

The process of the invention is a process for the preparation of a flowable aqueous solution of betaines of the general formula 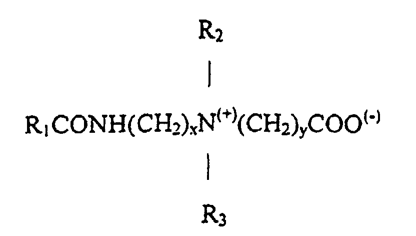 wherein
R₁ is the alkyl portion of a fatty acid with 6 to 24, preferably 6 to 18 carbon atoms ;
R₂ and R₃ are the same or different and represent alkyl with 1 to 5 carbon atoms ;
x is 1, 2 , 3, 4 or 5, preferably 2 or 3, and
y is 1,2 or 3,
in which a fatty acid amide of formula

R₁CONH(CH₂)ₓNR₂R₃

is quaternized with at least a ω-halogenoalkylcarboxylic acid of formula X(CH₂)_{y}COOH with X a halogen atom or a salt thereof, at least partly in an aqueous medium having a pH greater than 4 and a solid content lower than 90% by weight,
in which at the end of the quaternization reaction, the solid content of the betaine solution is lower than 70% by weight or the solid content of the betaine solution is adjusted at the end of the quaternization reaction to less than 70% by weight, and
in which at the end of the quaternization reaction the pH of the aqueous betaine solution is comprised between 4 and 8.5 or after the end of the quaternization reaction, the pH of the aqueous solution of betaine is adjusted to a value comprised between 4 and 8.5.
characterized in that the quaternization reaction is at least partly carried out in presence of an amount of a compound of formula 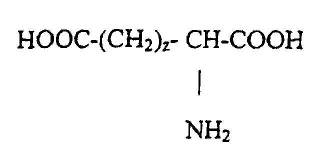 with z an integer from 1 to 10
or a salt thereof or the product of reaction thereof with a ω-halogenoalkylcarboxylic acid of formula X(CH₂)_{y}COOH with X a halogen atom or a salt thereof, said amount being sufficient for ensuring that the betaine solution with a solid content of 45%, advantageously of at least 45%, preferably of about 50% or the betaine solution concentrated to a solid content of 45%, advantageously of at least 45%, preferably of about 50% is flowable at a temperature of 10°C, advantageously at a temperature lower than 10°C.

Knowing that a betaine solution with a solid content of 47% ( prepared by quaternization in presence of 1% glutamic acid which has reacted with the halogenoalkylcarboxylic acid) is fluid at a temperature of less than 10°C, the man skilled in the art can determine by trial experiment an efficient amount of compound required. For example, the man skilled in the art can add to the said betaine solution further betaine and water so as to determine whether the glutamic acid content can be lowered while still having a fluid betaine solution at 10°C. The man skilled in the art can also concentrate the betaine solution for determining whether the concentrated betaine solution with a solid content of 50-60% is still fluid at 10°C.

Advantageously, the quaternization reaction is at least partly carried out in presence of a compound of formula 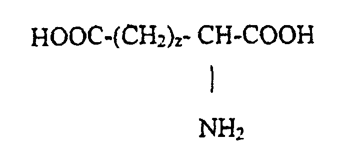 with z an integer from 1 to 6
or a salt thereof or the product of reaction thereof with a ω-halogenoalkylcarboxylic acid of formula X(CH₂)_{y}COOH with X a halogen atom or a salt thereof.

Preferably, the quaternization reaction is at least partly carried out in presence of a compound of formula 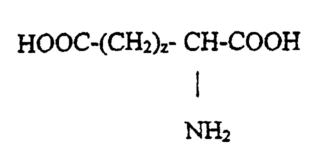 with z equal to 2
or a salt thereof or the product of reaction thereof with a ω-halogenoalkylcarboxylic acid of formula X(CH₂)_{y}COOH with X a halogen atom or a salt thereof.

According to an embodiment, at least 20% of the quaternization reaction, advantageously at least 50%, preferably at least 60%, most preferably at least 80% of the quaternization reaction, possibly substantially the complete quaternization reaction is carried out in presence of a compound of formula 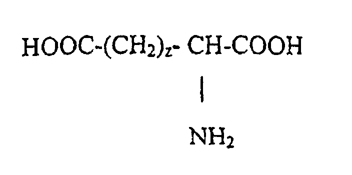 with z an integer from 1 to 10, advantageously from 1 to 6, preferably equal to 2, or a salt thereof or the product of reaction thereof with a ω-halogenoalkylcarboxylic acid of formula X(CH₂)_{y}COOH with X a halogen atom or a salt thereof.

The quaternization reaction is advantageously carried out in at least two steps, namely a first step in which a part of the amidoamine is reacted with an excess of ω-halogenoalkylcarboxylic acid of formula X(CH₂)_{y}COOH with X a halogen atom or a salt thereof, and a second step in which the remaining amidoamine is added to the betaine solution prepared in the first step. The compound of formula IV is advantageously already present in the quaternization of the first reaction step.

Advantageously, the quaternization reaction is carried out in an aqueous medium with a pH comprised between 6 and 11.

According to an embodiment of the process of the invention, at the end of the quaternization reaction, the solid content of the betaine solution is lower than 60%, advantageously less than 55% by weight, preferably less than about 50% by weight, or is adjusted to less than 60% by weight, advantageously less than 55% by weight, preferably less than about 50% by weight. The solid content can be increased or adjusted for example by evaporation or by addition of water.

According to another embodiment of the process of the invention, at the end of the quaternization reaction, the solid content of the betaine solution is comprised between 45 and 60%, preferably between 45 and 55% or the solid content of the betaine solution is adjusted at the end of the quaternization reaction between 45 and 60%, preferably between 45 and 55% by weight. The solid content can be increased or adjusted for example by evaporation.

According to another possible embodiment, at the end of the quaternization reaction the pH of the aqueous betaine solution is comprised between 4 and 7, advantageously between 4 and 6, most preferably between 4.5 and 6, or after the end of the quaternization reaction, the pH of the aqueous solution of betaines is adjusted to a value comprised between 4 and 7, advantageously between 4 and 6, most preferably between 4.5 and 6.

According to an advantageous embodiment, the quaternization reaction is at least partly carried out in presence of an organic acid or a salt thereof and/or after the end of the quaternization reaction, an organic acid or a salt thereof is added to the betaine solution for adjusting the pH. Possibly other additional additives can be used. However, in order to achieve the highest purity as possible, it is preferable to avoid such additional additives.

When an organic acid is used, the acid is advantageously a hydroxy carboxylic organic acid or a salt thereof, preferably citric acid or a salt thereof, especially the sodium salt thereof.

According to a detail of a process according to the invention, at the end of the quaternization reaction, the pH of the betaine solution is lowered to less than 4.6 by adding a sufficient amount of an organic acid, and in that thereafter, the pH of the betaine solution is increased to a value comprised between 4.6 and 6 by adding sodium hydroxide.

Examples of process according to the invention and of comparative processes will now be disclosed.

### EXAMPLE 1

### Preparation of the amido amine

The amido amine was prepared by reacting methylcocoate or a coconut oil (hydrogenated coconut oil) and 3-dimethyl amino propylamine. This reaction is well kown by the skilled art worker.

For example, the amido amine can be prepared as follows :
4160 kg of 3-Dimethyl amino propylamine was put in a reaction vessel and purged with nitrogen for 5 minutes. 9815 kg of hydrogenated coconut oil is added. The mixture is heated upto 30°C. Then 485 kg of sodium methylate solution (30% in methanol) is added to the reactor as catalyst.
The temperature of the reaction is then kept around 100°C during 2 hours.

After said reaction time, the amidoamine contained no free ester and no free amine (i.e. no free or unreacted amino amine).

### Preparation of the betaine by quaternization of the amidoamine

In a reactor, 416 kg demineralized water and 4.0 kg glutamic acid (i.e. a compound of formula HOOC-CH₂-CH₂-C(NH₂)H-COOH) are stirred together upto the complete dissolution of the acid. The pH of the mixture is then adjusted to about 9 by adding sodium hydroxide (as a 50% sodium hydroxide solution). 21.7 kg Glycerin was then added to the mixture. After stirring, 246 kg of the amidoamine was added. Then, mono chloro acetic acid ( with a di chloro acetic acid content of less than 70ppm) was added up to reaching a pH of 8.5. Thereafter, 63.5 kg sodium salt of mono chloro acetic acid (with a content of sodium salt of di chloro acetic acid of less than 70 ppm) was slowly added. After adding the said sodium salt of mono chloro acetic acid, the mixture was thick. The quatemization reaction started. As soon as the reaction mixture was fluid, mono chloro acetic acid was further slowly added. In total, 27 kg of mono chloro acetic acid was added. During said addition of mono chloro acetic acid, sodium hydroxide (as a 50% aqueous solution) was added so as to adjust the pH to 9. After adding the necessary amount of mono chloro acetic acid, the temperature of the reaction mixture was maintained to 90-95°C during 1 hour. The pH of the reaction mixture was maintained to about 9 by adding sodium hydroxide. After a reaction time of about 8 hours, the mono chloro acetic acid content of the reaction mixture was analyzed. In case, the content of mono chloro acetic acid was higher than 10 ppm, the reaction was further continued and possibly some amidoamine was added for having the complete reaction of the mono chloro acetic acid.

The pH of the betaine solution was lowered to 5 by adding citric acid.

The aqueous betaine solution prepared had a solid content of about 47% (sodium chloride content : about 6%, glycerin content : about 2.6%. betaine content: about 39%) and a pH of about 9. The free fatty acid content of the aqueous betaine solution was determined by Gas chromatography (GC-FID) without derivatisation of the fatty acids into the corresponding Fatty acid methyl esters. The determined free fatty acid measured according to said method was 1.2%. The mono chloro acetic acid content of the solution was less than 10 ppm, while due to the low amount of di chloro acetic acid present in the mono chloro acetic acid, the di chloro acetic acid content was less than 10 ppm.

The betaine solution was flowable at a temperature of 20°C, as well as at a temperature of 4°C.

### COMPARATIVE EXAMPLE 2

Example 1 was repeated, except that no glutamic acid was used.

The betaine solution having a slid content of about 46%, a pH of 5 and a free fatty acid content of 1.2% was not flowable at 20°C.

### COMPARATIVE EXAMPLE 3

Example 2 was repeated, except that at the end of the quaternization reaction, 4.0kg of glutamic acid was added, before adjusting the pH to 5 with citric acid.

The betaine solution was not fluid at 20°C.

### COMPARATIVE EXAMPLE 4

Example 2 was repeated, except that after the pH adjusting step to 5 by using citric. 4.0 kg glutamic acid was added. For keeping the pH to 5, NaOH was further added.

The betaine solution was not flowable at 20°C.

### EXAMPLE 5

Example 1 was repeated, except that the glutamic acid was added to the amidoamine after its preparation.

The betaine solution was flowable at a temperature lower than 10°C.

### EXAMPLE 6

Example 1 was repeated, except that a larger amount of demineralized water (600kg) was used for preparing a betaine solution with a solid content of about 40%.

The so prepared betaine solution was concentrated (by water evaporation) up to a solid content of 45%. The so prepared betaine solution was fluid at a temperature of less than 10°C.

### EXAMPLE 7

Example 1 was repeated, except that at the end of the quaternization reaction, the pH was adjusted to 4.5. The betaine solution was fluid at a temperature of less than 2°C.

### EXAMPLE 8

Example 1 was repeated, except that 8.0 kg glutamic acid was used and that the pH was finally adjusted to 5.2. The betaine solution (containing about 1% reaction product of glutamic acid with mono dichloro acetic acid) was fluid at a temperature of less than -5°C.

### EXAMPLE 9

Example 8 was repeated, except that a smaller amount of water was used for obtaining a solid content of 50% (betaine content of about 40-42%) and that the pH was adjusted to 4.9.

The betaine solution was fluid at a temperature of less than -10°C.

### EXAMPLES 10 TO 17.

Example 1 was repeated, except that the amount of glutamic acid used was adjusted for obtaining a betaine solution with a solid content of about 47% and glutamic acid content of respectively 0.4%, 0.6%, 0.7%, 0.75%, 0.8%, 1.2%, 1.5%, 2.0%.

### EXAMPLE 18

Example 1 was repeated, except that after the end of the quaternization, the pH was lowered to 4.5 by adding citric acid and thereafter adjusted to 5.0 by sodium hydroxide (50% solution).

### EXAMPLE 19

In said example, the amido amine was prepared as in example 1.

Hot demineralized water was put in a reactor.

The glutamic acid was added to the water. After the complete dissolution of the glutamic acid. the pH was adjusted to 8.5-9.5 by adding sodium hydroxide (50% aqueous solution). Glycerine is possibly added.

A first part of amidoamine (for example at least 50% of the amidoamine required for the preparation of the betaine, preferably 60 to 80% of the betaine) is then added to the solution. Mono chloro acetic acid (preferably containing less than 70ppm dichloroacetic acid) was added up to adjusting the pH to 8.5.

Sodium mono chloroacetate (preferably containing less than 70ppm dichloroacetate) was then added.

Mono chloroacetic acid was further used as soon as the solution was still fluid. When adding said acid, the pH was adjusted to 8.5-9.5 by adding sodium hydroxide (50% aqueous solution).

The temperature of the reaction is maintained to about 90°C. During said reaction, the pH is maintained to about 9 by adding sodium hydroxide (50% aqueous solution). The pressure in the reactor during the said reaction was above the atmospheric pressure (for example about 1.2 10⁵ Pa to 2 10⁵Pa, the actual pressure depending from the reaction temperature).

The amount of amidoamine still required for the preparation of the betaine is then added. During said reaction, the pH is maintained to about 9 by adding sodium hydroxide (50% aqueous solution).

After one hour reaction, the temperature was maintained to about 100°C for one hour.
The temperature is then lowered to about 60°C and the pressure of the reactor is reduced to the atmospheric pressure. The pH of the betaine is adjusted to a value between 4 and 8, preferably between 4.5 and 7, most preferably between 4.5 and 6.

The betaine solution is filtered by meas of a filter with opening having a diameter of 300 µm (or possibly less than 300µm).

When preparing the betaine according to a two step process, the same amidoamine or different amidoamine can be used in each quaternization step. For example a first amidoamine is quaternized during the first step, while a second amidoamine is added to the betaine solution prepared in the first step. In such a case, it is advantageous to first quaternize the amido amine having the radical R₁ with the highest amount of carbon atoms or with, in average, the highest amount of carbon atoms, and then to add for quaternization the amido amine having a radical R₁ with less carbon atoms or with in average less carbon atoms, than the first used amidoamine.

In the process of the invention, it is preferred to use glutamic acid or salt thereof having a high purity, for example a purity of at least 99% by weight (food grade), as said glutamic acid or salt is not toxic and is a food additive.

So, when using mono chloro acetic acid or a salt thereof with a high purity, for example containing di chloro acetic acid or salt thereof, it is possible to obtain without any heat treatment at a temperature of 115°C or more, a betaine solution with a solid content of 50% containing less than 10ppm dichloroacetic acid or salt thereof.

In case, the fluidity at 10°C of a composition prepared according to the process has to be increased, the pH of the composition can be lowered, for example between 4 and 5 and/or a larger amount of compound of formula IV can be used in the preparation step of the betaine and/or citric acid or sodium citrate can be added to the composition.

## Claims

1. Process for the preparation of a flowable aqueous solution of betaines of the general formula 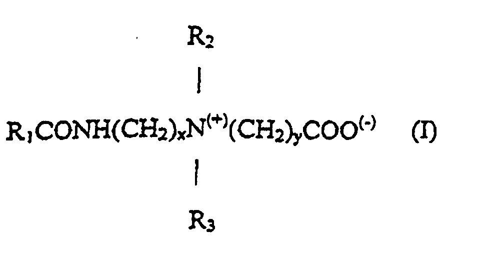 wherein
R₁ is the alkyl portion of a fatty acid with 6 to 24 carbon atoms, preferably with 6 to 18 carbon atoms ;
R₂ and R₃ are the same or different and represent alkyl with 1 to 5 carbon atoms ;
x is equal to 1,2,3,4 or 5, but is preferably equal to 2 or 3, and
y is 1,2 or 3,
in which a fatty acid amide of formula
R₁CONH(CH₂)ₓNR₂R₃ (II)
is quaternized with at least a co-halogenoalkylcarboxylic acid of formula X(CH₂)_{y}COOH (III) with X a halogen atom or a salt thereof, at least partly in an aqueous medium having a pH greater than 4 and a solid content lower than 90%.
in which at the end of the quaternization reaction, the solid content of the betaine solution is lower than 70% or the solid content of the betaine solution is adjusted at the end of the quaternization reaction to less than 70%, and
in which at the end of the quaternization reaction the pH of the aqueous betaine solution is comprised between 4 and 8.5 or after the end of the quaternization reaction, the pH of the aqueous solution of betaine is adjusted to a value comprised between 4 and 8.5,
**characterized in that** the quaternization reaction is at least partly carried out in presence of an amount of a compound of formula 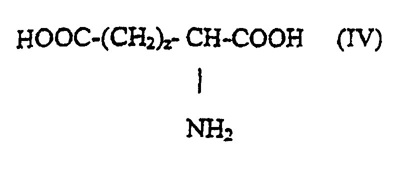 with z an integer from 1 to 10
or a salt thereof or the product of reaction of said acid or salt with a ω-halogenoalkylcarboxylic acid of formula X(CH₂)_{y}COOH with X a halogen atom or a salt thereof, said amount being sufficient for ensuring that the betaine solution with a solid content of 45%, advantageously of at least 45%, preferably of about 50% or the betaine solution concentrated to a solid content of 45%, advantageously of at least 45%, preferably of about 50% is flowable at a temperature of 10°C, advantageously at a temperature lower than 10°C.

2. The process of claim 1, **characterized in that** the quaternization reaction is at least partly carried out in presence of a compound of formula (IV) 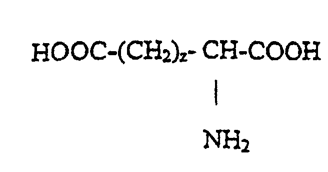 with z an integer from 1 to 6 ;
or a salt thereof or the product of reaction of said acid or salt with a ω-halogenoalkylcarboxylic acid of formula X(CH₂)_{y}COOH with X a halogen atom or a salt thereof.

3. The process of claim 1, **characterized in that** the quaternization reaction is at least partly carried out in presence of a compound of formula (IV) 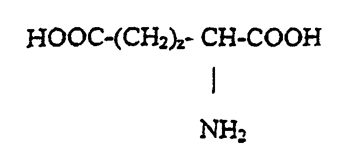 with z equal to 2,
or a salt thereof or the product of reaction of said acid or salt with a ω-halogenoalkylcarboxylic acid of formula X(CH2)yCOOH with X a halogen atom or a salt thereof.

4. The process of claim 1, **characterized in that** at least 20% of the quaternization reaction, preferably at least 50% of the quaternization reaction is carried out in presence of a compound of formula (IV) 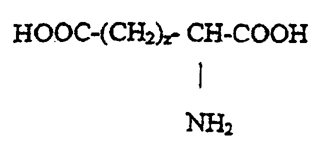 with z an integer from 1 to 10, advantageously from 1 to 6, preferably equal to 2, or a salt thereof or the product of reaction of said acid or salt with a ω-halogenoalkylcarboxylic acid of formula X(CH2)yCOOH with X a halogen atom or a salt thereof.

5. The process of claim 1, **characterized in that** at least 60% of the quaternization reaction. preferably at least 80% of the quaternization reaction is carried out in presence of a compound of formula 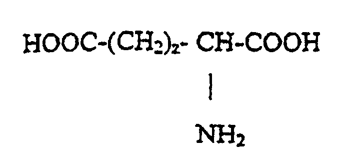 with z an integer from 1 to 10, advantageously from 1 to 6, preferably equal to 2, or a salt thereof or the product of reaction of said acid or salt with a ω-halogenoalkylcarboxylic acid of formula X(CH₂)_{y}COOH with X a halogen atom or a salt thereof.

6. The process of claim 1, **characterized in that** substantially all the quaternization reaction is carried out in presence of a compound of formula 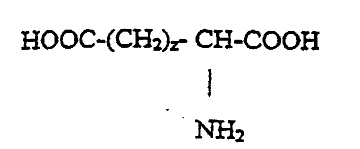 with z an integer from 1 to 10, advantageously from 1 to 6, preferably equal to 2. or a salt thereof or the product of reaction of said acid or salt with a ω-halogenoalkylcarboxylic acid of formula X(CH2)yCOOH with X a halogen atom or a salt thereof.

7. The process of any one of the preceding claims, **characterized in that** the quaternization reaction is carried out in an aqueous medium with a pH comprised between 6 and 11.

8. The process of any one of the preceding claims, **characterized in that** at the end of the quaternization reaction, the solid content of the betaine solution is comprised between 45 and 60% or the solid content of the betaine solution is adjusted at the end of the quaternization reaction between 45 and 60%.

9. The process of any one of the preceding claims, **characterized in that** at the end of the quaternization reaction the pH of the aqueous betaine solution is comprised between 4 and 7 or in which after the end of the quaternization reaction, the pH of the aqueous solution of betaines is adjusted to a value comprised between 4 and 7.

10. The process of any one of the preceding claims, **characterized in that** at the end of the quaternization reaction the pH of the aqueous betaine solution is comprised between 4 and 6 or in which after the end of the quaternization reaction, the pH of the aqueous solution of betaines is adjusted to a value comprised between 4 and 6.

11. The process of any one of the preceding claims, **characterized in that** the quaternization reaction is at least partly carried out in presence of an organic acid or a salt thereof and/or after the end of the quaternization reaction, an organic acid or a salt thereof is added to the betaine solution for adjusting the pH.

12. The process of the preceding claim, **characterized in that** the organic acid is citric acid or a salt thereof, especially the sodium salt thereof.

13. The process of any one of the preceding claims, **characterized in that** at the end of the quaternization reaction, the pH of the betaine solution is lowered to less than 4.6 by adding a sufficient amount of an organic acid, and **in that** thereafter, the pH of the betaine solution is increased to a value comprised between 4.6 and 6 by adding sodium hydroxide.

14. The process of any one of the preceding claims, **characterized in that** at the end of the quaternization reaction, the solid content of the betaine solution is lower than 60%, advantageously less than than 55%, preferably less than about 50%, or the solid content of the betaine solution is adjusted at the end of the quaternization reaction to less than 60%, advantageously less than 55%, preferably less than about 50%.

15. The process of anyone of the preceding claims, **characterized in that** the quaternization reaction is at least partly carried out in presence of the reaction product of a compound of formula 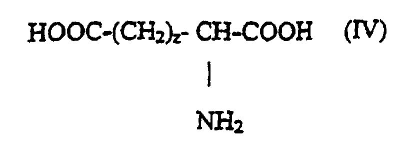 with z an integer from 1 to 10
or a salt thereof, with a ω-halogenoalkylcarboxylic acid of formula X(CH₂)_{y}COOH with X a halogen atom or a salt thereof.

## Patentansprüche

1. Ein Verfahren zur Herstellung einer fließfähigen wäßrigen Lösung von Betainen der allgemeinen Formel 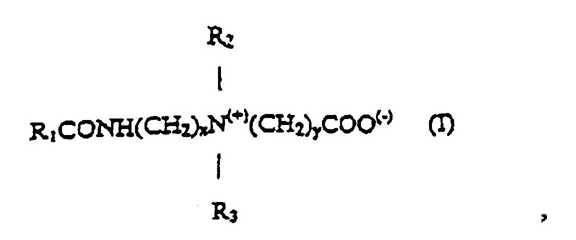 wobei
R₁ der Alkylteil einer Fettsäure mit 6 bis 24 Kohlenstoffatomen, bevorzugt mit 6 bis 18 Kohlenstoffatomen ist;
R₂ und R₃ dieselben oder unterschiedliche sind und Alkyl mit 1 bis 5 Kohlenstoffatomen darstellen;
x gleich 1, 2, 3, 4 oder 5 ist, aber bevorzugt gleich 2 oder 3 ist, und
y 1, 2 oder 3 ist,
bei dem ein Fettsäureamid der Formel
R₁CONH(CH₂)ₓNR₂R₃ (II)
mit wenigstens einer ω-Halogenalkylcarbonsäure der Formel X(CH₂)_{y}COOH (III), wobei X ein Halogenatom ist, oder einem Salz davon quaternisiert wird, wenigstens teilweise in einem wäßrigen.Medium mit einem pH größer als 4 und einem Feststoffgehalt, der niedriger als 90% ist,
bei dem am Ende der Quaternisierungsreaktion der Feststoffgehalt der Betainlösung niedriger als 70% ist oder der Feststoffgehalt der Betainlösung am Ende der Quaternisierungsreaktion auf weniger als 70% eingestellt wird, und
bei dem am Ende der Quaternisierungsreaktion der pH der wäßrigen Betainlösung im Bereich von 4 bis 8,5 liegt oder nach dem Ende der Quaternisierungsreaktion der pH der wäßrigen Lösung von Betain auf einen Wert im Bereich von 4 bis 8,5 eingestellt wird,
**dadurch gekennzeichnet, daß** die Quaternisierungsreaktion wenigstens teilweise in der Anwesenheit einer Menge einer Verbindung der Formel 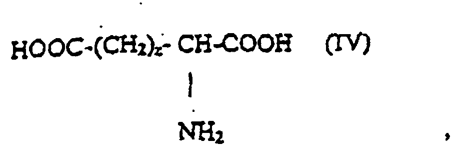 wobei z eine ganze Zahl von 1 bis 10 ist, oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird, wobei die Menge ausreicht, um sicherzustellen, daß die Betainlösung mit einem Feststoffgehalt von 45% vorteilhafterweise von wenigstens 45%, bevorzugt von ungefähr 50%, oder die Betainlösung, die auf einen Feststoffgehalt von 45%, vorteilhafterweise von wenigstens 45%, bevorzugt von ungefähr 50%, aufkonzentriert wurde, bei einer Temperatur von 10°C fließfähig ist, vorteilhafterweise bei einer Temperatur, die niedriger als 10°C ist.

2. Verfahren nach Anspruch 1, **dadurch gekennzeichnet, daß** die Quaternisierungsreaktion wenigstens teilweise in Anwesenheit einer Verbindung der Formel (IV) 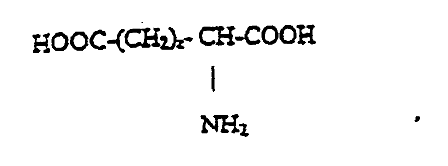 wobei z eine ganze Zahl von 1 bis 6 ist,
oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird.

3. Verfahren nach Anspruch 1, **dadurch gekennzeichnet, daß** die Quaternisierungsreaktion wenigstens teilweise in Anwesenheit einer Verbindung der Formel (IV) 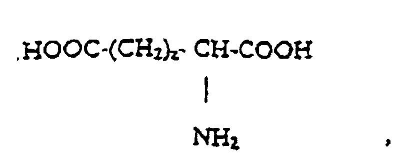 wobei z gleich 2 ist,
oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird.

4. Verfahren nach Anspruch 1, **dadurch gekennzeichnet, daß** wenigstens 20% der Quaternisierungsreaktion, bevorzugt wenigstens 50% der Quaternisierungsreaktion, in Anwesenheit einer Verbindung der Formel (IV) 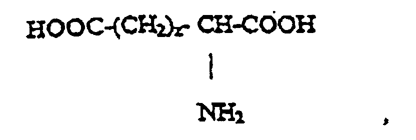 wobei z eine ganze Zahl von 1 bis 10, vorteilhafterweise von 1 bis 6, bevorzugt gleich 2 ist, oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird.

5. Verfahren nach Anspruch 1, **dadurch gekennzeichnet, daß** wenigstens 60% der Quaternisierungsreaktion, bevorzugt wenigstens 80% der Quaternisierungsreaktion, in Anwesenheit einer Verbindung der Formel 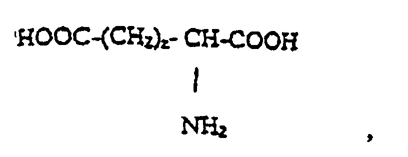 wobei z eine ganze Zahl von 1 bis 10, vorteilhafterweise von 1 bis 6, bevorzugt gleich 2 ist, oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird.

6. Verfahren nach Anspruch 1, **dadurch gekennzeichnet, daß** im wesentlichen die gesamte Quaternisierungsreaktion in Anwesenheit einer Verbindung der Formel 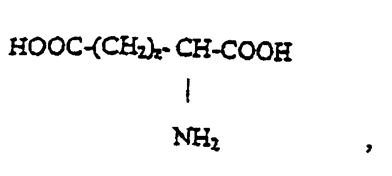 wobei z eine ganze Zahl von 1 bis 10, vorteilhafterweise von 1 bis 6, bevorzugt gleich 2 ist, oder eines Salzes davon oder des Produkts einer Reaktion der Säure oder Salz mit einer ω-Halogenalkylcarbonsäure der Formel X(CH₂)_{y}COOH, wobei X ein Halogenatom ist, oder einem Salz davon durchgeführt wird.

7. Verfahren nach einem der vorangehenden Ansprüche, **dadurch gekennzeichnet, daß** die Quaternisierungsreaktion in einem wäßrigem Medium mit einem pH, der im Bereich von 6 bis 11 liegt, durchgeführt wird.

8. Verfahren nach einem der vorangehenden Ansprüche, **dadurch gekennzeichnet, daß** am Ende der Quaternisierungsreaktion der Feststoffgehalt der Betainlösung in einem Bereich von 45 bis 60% liegt oder der Feststoffgehalt der Betainlösung am Ende der Quaternisierungsreaktion auf zwischen 45 und 60% eingestellt wird.

9. Verfahren nach einem der vorangehenden Ansprüche, **dadurch gekennzeichnet, daß** am Ende der Quaternisierungsreaktion der pH der wäßrigen Betainlösung im Bereich von 4 bis 7 liegt oder bei dem nach dem Ende der Quaternisierungsreaktion der pH der wäßrigen Lösung von Betainen auf einen Wert im Bereich von 4 bis 7 eingestellt wird.

10. Verfahren nach einem der vorangehenden Ansprüche, **dadurch gekennzeichnet, daß** am Ende der Quaternisierungsreaktion der pH der wäßrigen Betainlösung im Bereich von 4 bis 6 liegt, oder bei dem nach dem Ende der Quaternisierungsreaktion der pH der wäßrigen Lösung von Betainen auf einen Wert im Bereich von 4 bis 6 eingestellt wird.

11. Verfahren nach einem der vorangehenden Ansprüche, **dadurch gekennzeichnet, daß** die Quaternisierungsreaktion wenigstens teilweise in Anwesenheit einer organischen Säure oder eines Salzes davon durchgeführt wird, und/oder nach dem Ende der Quaternisierungsreaktion eine organische Säure oder ein Salz davon zur Betainlösung zum Einstellen des pH zugegeben wird.

12. Verfahren nach dem vorangehenden Anspruch, **dadurch gekennzeichnet, daß** die organische Säure Citronensäure oder ein Salz davon, insbesondere das Natriumsalz ist

13. , Verfahren nach einem der vorangehenden Ansprüche, **dadurch gekennzeichnet, daß** am Ende der Quaternisierungsreaktion der pH der Betainlösung durch Zugabe einer ausreichenden Menge an organischer Säure auf weniger als 4,6 gesenkt wird, und daß danach der pH der Betainlösung durch Zugabe von Natriumhydroxid auf einen Wert zwischen 4,6 und 6 erhöht wird.

14. Verfahren nach einem der vorangehenden Ansprüche, **dadurch gekennzeichnet, daß** am Ende der Quaternisierungsreaktion der Feststoffgehalt der Betainlösung niedriger als 60%, vorteilhafterweise weniger als 55%, bevorzugt weniger als ungefähr 50% ist, oder daß der Feststoffgehalt der Betainlösung am Ende der Quaternisierungsreaktion auf weniger als 60%, vorteilhafterweise weniger als 55%, bevorzugt weniger als ungefähr 50% eingestellt wird.

15. Verfahren nach einem der vorangehenden Ansprüche, **dadurch gekennzeichnet, daß** die Quaternisierungsreaktion wenigstens teilweise in Anwesenheit des Reaktionsprodukts einer Verbindung der Formel 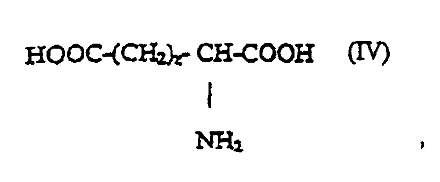 wobei z eine ganze Zahl von 1 bis 10 ist,
oder eines. Salzes davon mit einer ω-Halogenalkylcarbonsäure der Formel X(CH₂)_{y}COOH, wobei X ein Halogenatom ist, oder einem Salz davon durchgeführt wird.

## Revendications

1. Procédé pour la préparation d'une solution aqueuse de bétaïnes apte à l'écoulement de formule générale 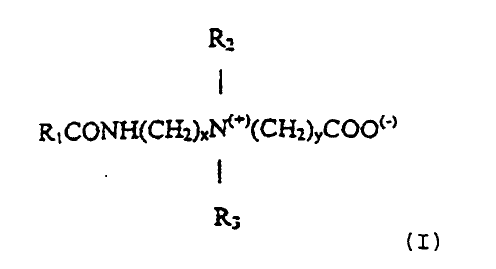 dans laquelle
R₁ est la portion d'alkyle d'un acide gras avec 6 à 24 atomes de carbone, de préférence 6 à 18 atomes de carbone ;
R₂ et R₃ sont identiques ou différents et représentent un alkyle avec 1 à 5 atomes de carbone ; x est égal à 1, 2, 3, 4 ou 5, mais est de préférence égal à 2 ou 3, et y est 1, 2 ou 3,
dans lequel un amide d'acide gras de formule
R₁CONH (CH₂)ₓNR₂R₃ (II)
est quaternisé avec au moins un acide ω-halogénoalkylcarboxylique de formule X(CH₂)_{y}COOH, X étant un atome d'halogène ou un sel de celui-ci, au moins partiellement dans un milieu aqueux ayant un pH plus grand que 4 et une teneur en solide inférieure à 90%,
dans lequel à la fin de la réaction de quaternisation, la teneur en solide de la solution de bétaïne est inférieure à 70% ou la teneur en solide de la solution de bétaïne est ajustée à la fin de la réaction de quaternisation à moins de 70%, et
dans lequel à la fin de la réaction de quaternisation le pH de la solution aqueuse de bétaïne est compris entre 4 et 8,5 ou après la fin de la réaction de quaternisation, le pH de la solution aqueuse de bétaïne est ajusté à une valeur comprise entre 4 et 8,5,
**caractérisé en ce que** la réaction de quaternisation est au moins partiellement effectuée en présence d'une quantité d'un composé de formule 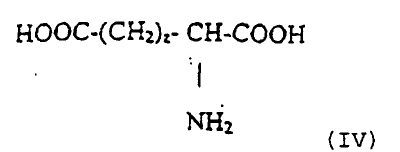 z étant un entier de 1 à 10
ou un sel de celui-ci ou le produit de la réaction dudit acide ou dudit sel avec un acide ω-halogénoalkyl-carboxylique de formule X(CH₂)_{y}COOH, X étant un atome d'halogène ou un sel de celui-ci, ladite quantité étant suffisante pour être sûr que la solution de bétaïne avec une teneur en solide de 45%, avantageusement d'au moins 45%, de préférence d'environ 50% ou la solution de bétaïne concentrée à une teneur en solide de 45%, avantageusement d'au moins 45%, de préférence d'environ 50% est apte à l'écoulement à une température de 10°C, avantageusement à une température inférieure à 10°C.

2. Procédé selon la revendication 1, **caractérisé en ce que** la réaction de quaternisation est au moins partiellement effectuée en présence d'un composé de formule (IV) 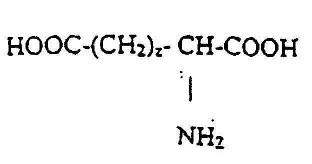 z étant un entier de 1 à 6 ;
ou un sel de celui-ci ou le produit de la réaction dudit acide ou dudit sel avec un acide ω-halogénoalkyl-carboxylique de formule X(CH₂)_{y}COOH, X étant un atome d'halogène ou un sel de celui-ci.

3. Procédé selon la revendication 1, **caractérisé en ce que** la réaction de quaternisation est au moins partiellement effectuée en présence d'un composé de formule (IV) 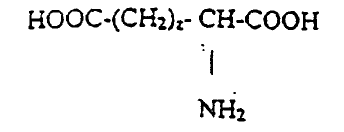 avec z égal à 2,
ou un sel de celui-ci ou le produit de la réaction dudit acide ou dudit sel avec un acide ω-halogénoalkyl-carboxylique de formule X(CH₂)_{y}COOH, X étant un atome d'halogène ou un sel de celui-ci.

4. Procédé selon la revendication 1, **caractérisé en ce qu'**au moins 20% de la réaction de quaternisation, de préférence au moins 50% de la réaction de quaternisation est effectuée en présence d'un composé de formule (IV) 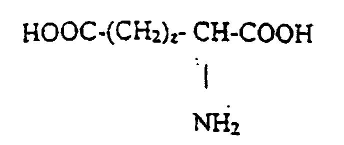 z étant un entier de 1 à 10, avantageusement de 1 à 6, de préférence égal à 2,
ou un sel de celui-ci ou le produit de la réaction dudit acide ou du sel avec un acide ω-halogénoalkyl-carboxylique de formule X(CH₂)_{y}COOH, X étant un atome d'halogène ou un sel de celui-ci.

5. Procédé selon la revendication 1, **caractérisé en ce qu'**au moins 60% de la réaction de quaternisation, de préférence au moins 80% de la réaction de quaternisation est effectuée en présence d'un composé de formule 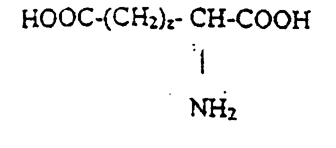 z étant un entier de 1 à 10, avantageusement de 1 à 6, de préférence égal à 2,
ou un sel de celui-ci ou le produit de la réaction dudit acide ou du sel avec un acide ω-halogénoalkyl-carboxylique de formule X(CH₂)_{y}COOH, X étant un atome d'halogène ou un sel de celui-ci.

6. Procédé selon la revendication 1, **caractérisé en ce que** substantiellement toute la réaction de quaternisation est effectuée en présence d'un composé de formule 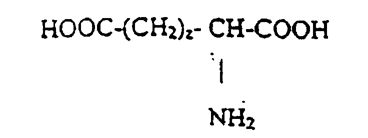 z étant un entier de 1 à 10, avantageusement de 1 à 6, de préférence égal à 2,
ou un sel de celui-ci ou le produit de la réaction dudit acide ou du sel avec un acide ω-halogénoalkyl-carboxylique de formule X(CH₂)_{y}COOH, X étant un atome d'halogène ou un sel de celui-ci.

7. Procédé selon l'une quelconque des revendications précédentes, **caractérisé en ce que** la réaction de quaternisation est effectuée dans un milieu aqueux avec un pH compris entre 6 et 11.

8. Procédé selon l'une quelconque des revendications précédentes, **caractérisé en ce qu'**à la fin de la réaction de quaternisation, la teneur en solide de la solution de bétaïne est comprise entre 45 et 60% ou la teneur en solide de la solution de bétaïne est ajustée à la fin de la réaction de quaternisation entre 45 et 60%.

9. Procédé selon l'une quelconque des revendications précédentes, **caractérisé en ce qu'**à la fin de la réaction de quaternisation, le pH de la solution aqueuse de bétaïne est compris entre 4 et 7 ou dans lequel après la fin de la réaction de quaternisation, le pH de la solution aqueuse de bétaïnes est ajusté à une valeur comprise entre 4 et 7.

10. Procédé selon l'une quelconque des revendications précédentes, **caractérisé en ce qu'**à la fin de la réaction de quaternisation, le pH de la solution aqueuse de bétaïne est compris entre 4 et 6 ou dans lequel après la fin de la réaction de quaternisation, le pH de la solution aqueuse de bétaïnes est ajusté à une valeur comprise entre 4 et 6.

11. Procédé selon l'une quelconque des revendications précédentes, **caractérisé en ce que** la réaction de quaternisation est au moins partiellement effectuée en présence d'un acide organique ou d'un sel de celui-ci et/ou après la fin de la réaction de quaternisation, un acide organique ou un sel de celui-ci est ajouté à la solution de bétaïne pour ajuster le pH.

12. Procédé selon la revendication précédente, **caractérisé en ce que** l'acide organique est l'acide citrique ou un sel de celui-ci, spécialement le sel de sodium de celui-ci.

13. Procédé selon l'une quelconque des revendications précédentes, **caractérisé en ce qu'**à la fin de la réaction de quaternisation, le pH de la solution de bétaïne est abaissée à moins de 4,6 par l'addition d'une quantité suffisante d'un acide organique, et **en ce qu'**ensuite, le pH de la solution de bétaïne est augmenté à une valeur comprise entre 4,6 et 6 par l'addition d'hydroxyde de sodium.

14. Procédé selon l'une quelconque des revendications précédentes, **caractérisé en ce qu'**à la fin de la. réaction de quaternisation, la teneur en solide de la solution de bétaïne est inférieure à 60%, avantageusement inférieure à 55%, de préférence inférieure à environ 50%, ou la teneur en solide de la solution de bétaïne est ajustée à la fin de la réaction de quaternisation à moins de 60%, avantageusement moins de 55%, de préférence moins d'environ 50%.

15. Procédé selon l'une quelconque des revendications précédentes, **caractérisé en ce que** la réaction de quaternisation est au moins partiellement effectuée en présence du produit de la réaction d'un composé de formule 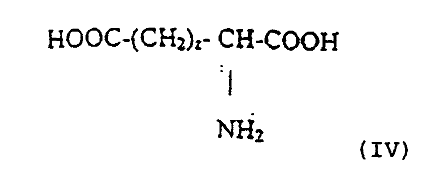 z étant un entier de 1 à 10
ou un sel de celui-ci, avec un acide ω-halogénoalkyl-carboxylique de formule X(CH₂)_{y}COOH, X étant un atome d'halogène ou un sel de celui-ci.
